Europäisches Patentamt

(19) European Patent Office

Office européen des brevets

(11) EP 0 691 203 A1

## (12) EUROPEAN PATENT APPLICATION

(43) Date of publication:
10.01.1996 Bulletin 1996/02

(51) Int. Cl.⁶: **B32B 31/04**, B32B 5/02, B32B 27/32, B29C 55/00, A61B 19/08, A61F 13/15

(21) Application number: 95108644.6

(22) Date of filing: 06.06.1995

(84) Designated Contracting States:
BE DE ES FR GB IT NL PT SE

(30) Priority: 06.06.1994 US 254207

(71) Applicant: KIMBERLY-CLARK CORPORATION
Neenah, Wisconsin 54956-0349 (US)

(72) Inventors:
• McCormack, Ann Louise
Cumming, GA 30131 (US)
• Garrett, Lance James, Jr.
Marietta, GA 30062 (US)

• English, Karen Lynn
Marietta, GA 30068 (US)

(74) Representative: Diehl, Hermann, Dr. Dipl.-Phys. et al
D-80639 München (DE)

Remarks:
A request for correction of error in Figure 3 has been filed pursuant to Rule 88 EPC. A decision on the request will be taken during the proceedings before the Examining Division (Guidelines for Examination in the EPO, A-V, 3.).

(54)     **Stretch-thinned film and nonwoven laminate and method for making same**

(57)     Disclosed herein is a stretch-thinned film and nonwoven laminate (10) as well as a process for making such a laminate in either a two or three-layer configuration. The film (12) has an effective gauge of 0.01397 mm (0.55 mils (0.00055 inches)) or less and a cross-machine direction to machine direction ratio of Elmendorf tear strength of 3.5 or greater. The resultant laminate (10) has a wide variety of uses which include components of health care related items such as sterile wrap, surgical drapes and gowns and body side liners and external covers for personal care absorbent products.

FIG. 3

EP 0 691 203 A1

## Description

The invention relates to stretch-thinned film and nonwoven laminates.

Films have been traditionally used to provide barrier properties in single-use items including, but not limited to, articles of clothing in general, protective apparel, healthcare related products including surgical drapes, gowns and sterile wrap and personal care absorbent products such as diapers, training pants, incontinence garments, sanitary napkins, bandages and the like. In personal care absorbent products such as infant diapers and adult incontinence products, films are used as the outer covers with the purpose of preventing body wastes from contaminating the clothing, bedding and other aspects of the surrounding environment of use. In the area of protective apparel including hospital gowns, films are used to prevent cross exchange of microorganisms between the wearer and the patient. These stand-alone films are usually 0.025 to 0.05 mm (one to two mils) in thickness and weigh approximately 23.73 to 50.85 g/m$^2$ (0.7 to 1.5 oz. per square yard). Polyolefin films are most commonly used in such areas and in their most basic forms have a cost of approximately four to six cents per 0.836 m$^2$ (square yard) based upon a 0.025 mm (one mil) thick film.

While these films can be effective barriers, they are not aesthetically pleasing because their surfaces are smooth and either feel slick or tacky. They are also visually flat and "plasticy" thereby making them less desirable in apparel applications and other uses where they are in contact with human skin. It would be more preferable if these items were more cloth-like from both a tactile and visual standpoint. For example, if infant diapers were more garment-like on their exterior surfaces they could obviate the need for additional clothing. Garment-like adult incontinence products could improve the self image of the incontinent individual. In addition, more garment-like isolation gowns would help the hospital environment feel less foreign and threatening to the patient.

Traditional non-breathable olefin films are available in lighter weights and are usually lower in cost per pound than the microporous films. While weights of 23.73 to 50.85 g/m$^2$ (0.7 to 1.5 osy) are common, weights as low as 13.56 g/m$^2$ (0.4 osy) are available. These lightweight films are often difficult to handle and do not have good strength properties.

Laminations of films and nonwovens have been used to create materials which are both impervious and somewhat cloth-like in appearance and texture. The outer covers on premium-priced diapers are but one example. Surgical gowns are another example. Such laminations are more expensive than stand-alone films and their usage has been limited to premium-priced products. Consequently, the need for more cost-effective, cloth-like, impervious barrier laminations has not been fully satisfied.

One reason such existing laminates are more costly than stand-alone films is because more materials are used, thereby resulting in heavier weight products. A second factor is the cost of the lamination process itself. Consequently, the ability to use lightweight materials and a simple, low-cost lamination process are desirable objectives.

A primary purpose of the film in such laminations is to provide barrier properties. In theory, extremely low weights (gauges) of film can meet this requirement. An object of the present invention is to provide extremely low weights and gauges of film in a nonwoven lamination that also can provide the aesthetic and durability properties desired in product usage. There is also a need for such laminates to be breathable so that they have the ability to transmit moisture vapor. Apparel made from laminations of these breathable or microporous films are more comfortable to wear and help keep the wearer's skin healthy by reducing the relative humidity underneath the apparel item. The more traditional, non-breathable films, however, may be preferred in protective apparel applications where chemical vapor penetration presents a contamination risk to the wearer.

An alternative to laminating a film to a nonwoven is to coat the nonwoven with the filming formulation. This process is known as extrusion coating. The extrusion coating process is capable of achieving low weight film coatings. The process relies on the residual heat within the thermally extruded film for adhesion to the nonwoven substrate. One problem with extrusion coating of extremely low weights of film is that the reduced polymer mass carries less heat and makes achieving good adhesion difficult. If adequate adhesion is not achieved, the coating tends to separate from the nonwoven during use so that it can become easily torn or ruptured. The extrusion coated film is not particularly strong since it has not been subjected to an orientation process. Film orientation processes are often used to increase the strength of stand-alone films.

When adequate adhesion of the film and nonwoven is achieved, the problem of durability during use is largely obviated. However, the structure is somewhat rigid and does not provide the tactile aesthetics that are desired in many applications where a low cost, cloth-like barrier laminate would be appropriate. With extrusion coated constructions a major reason for the stiffness of the overall laminate is the full area adhesion between the two layers. Full area bonding or adhesion provides more durable lamination but results in undesirable stiffness which tends to immobilize the fibers in the nonwoven so that it is not as pleasing to the touch. Consequently, there is a need to balance lamination strength and softness of the overall laminate.

Films may be down-gauged, that is reduced in weight and thickness, by drawing them in either the machine or cross direction. The film is usually heated prior to these drawing processes to make the film more plastic or malleable. This drawing or stretching also orients the molecular structure within the film which increases its strength and durability. The effect of down gauging is desired to reduce cost and the molecular orientation is desired to improve durability. Unfortunately, there are two problems with using stretch-thinned or oriented films for low-cost, cloth-like barriers. The stretch-thinning is an additional process that adds cost to the raw material. Stretching the film in the cross direction is particularly challenging because forces must be applied to the edges of the film to cause it to elongate. Tenter frames are commonly used. In contrast, stretch-thinning the film in the machine direction is relatively easy. It is only necessary to increase the draw, or speed ratio, between two rollers while the film is in the heated or plastic state. Machine-directionally stretched films are desired to achieve the lowest costs in down-gauged films because of process simplicity and the process speeds that can be achieved in comparison to cross-directionally stretch-thinned films.

There is a durability problem, however, with uni-directionally-stretch-thinned films, be it machine direction or cross-direction. Uni-directional stretching can achieve the down gauging objectives, but the molecular orientation occurs only in the stretched direction. This results in films that are easily torn or split along that dimension. For example, a machine-directionally oriented film has a propensity to split or tear along the machine direction. Also, the tensile characteristics of the film are dramatically increased in the machine direction, but the tensile strength in the cross-direction is significantly inferior to that of the machine direction.

These durability problems with uni-directionally stretched or oriented films are well known. Two approaches are commonly used to obviate the product durability problems resulting from these highly isotropic strength characteristics. The first is to stretch-orient the film in both the machine and cross direction. Films that have been biaxially stretched have more balanced strength properties. The second approach is to combine into a laminate one layer of machine directionally oriented film with one layer of cross-directionally oriented film. Neither approach is consistent with the cost objectives of the present invention because both entail the use of the cross directional stretching process. Further, the second approach doubles the minimum weight that can be achieved in the final laminate and consequently the cost of the material. There is therefore a need for a machine-directionally-stretched, lightweight film and nonwoven laminated using low-cost materials and processes that provides a laminate with both the cloth-like aesthetics and the in-use durability that are desired.

Therefore it is the object of the present invention to provide a stretch-thinned film and nonwoven laminate to over come the drawbacks of the known products. This object is solved by the process for forming the stretch-thinned film and nonwoven laminate according to independent claim 1 and the stretch-thinned film and nonwoven web according to independent claim 4. Further advantageous features, aspects and details of the invention are evident from the dependent claims, the description and the drawings. The claims are to understood as a first non-limiting approach to define the invention in general terms.

The present invention is directed to a process for making lightweight, low cost, cloth-like and optionally breathable film and nonwoven laminates wherein the film layer has been thinned to a very low gauge in the machine direction and subsequently laminated to one or more fibrous nonwoven web support layers to create a lightweight laminate which has a variety of uses including, but not limited to, a component or components for personal care absorbent products, articles of clothing and healthcare related items such as drapes, gowns and sterile wrap.

The present invention is directed to a stretch-thinned film and nonwoven laminate and a process for making such a laminate. The process involves sufficiently stretching a polyolefin-based film to cause the film to have an effective gauge of 0.01397 mm (0.55 mils) or less and a cross-machine direction to machine direction ratio of Elmendorf tear strengths of 3.5 or greater. Next a first fibrous nonwoven web support layer is bonded to the film to form the laminate. Desirably, the bonding of the laminate is in the form of discrete bond points with a maximum total bond area of about 60 percent. Optionally, a second fibrous nonwoven web support layer may be bonded to a surface of the film layer opposite that which is bonded to the first fibrous nonwoven web support layer.

The resultant two layer laminate has a ratio of cross-machine direction to machine direction Elmendorf tear strengths of less than 3.5 with the minimum machine direction Elmendorf tear strength being 100 grams or greater and the peak load cup crush value being less than 150 grams. With the three layer laminate the softness will not be as good but in any event the peak load cup crush value should be 300 grams or less.

Figure 1 is a perspective view of a stretch-thinned film and nonwoven laminate according to the present invention.

Figure 2 is a cross-sectional side view of another stretch-thinned film and nonwoven laminate according to the present invention.

Figure 3 is a schematic side view of a process for forming a stretch-thinned film and nonwoven laminate according to the present invention.

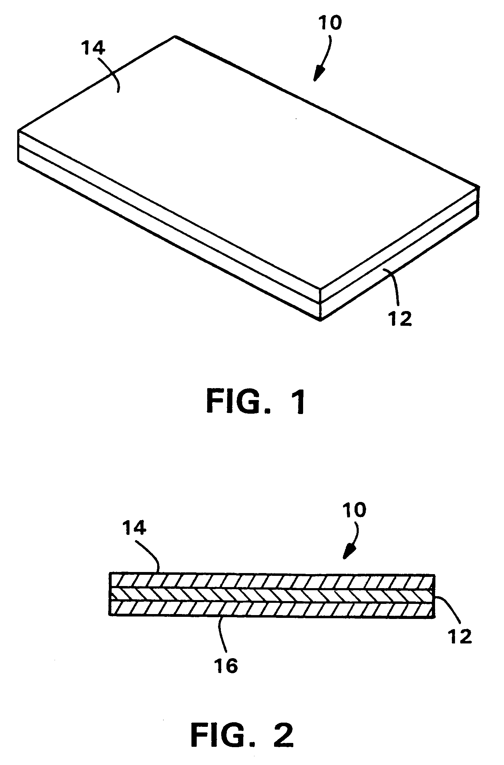

Referring to Fig. 1, there is shown a stretch-thinned film and nonwoven laminate 10 according to the present invention including a first film layer 12 and a first fibrous nonwoven web support layer 14. The film layer 12 can be made from either cast or blown film equipment and can be embossed if so desired. The film layer 12 can be made from any thermoplastic film which can be stretched in one direction such that the film gauge or thickness can be reduced from an initial gauge to an effective final gauge of 0.01397 mm (0.55 mils) or less. Generally, this stretching will occur in the machine direction. In addition, stretching may take place in the cross-machine direction provided, however, that the ratio of the cross-machine direction (CD) Elmendorf tear strength to the machine direction (MD) Elmendorf tear strength does not fall below about 3.5.

In some applications it may be desirable to make the overall laminate breathable. While most nonwovens are breathable, the same is not true with respect to films. Some films are made breathable by adding filler particles such as calcium carbonate to the film during the film-forming process. Once the particle-filled film has been formed, it is then either stretched or crushed to create pathways through the film, thereby making the film breathable. This too can add an additional step to the overall process and increase the price of the resultant material. Other films, such as a film sold under the trade designation FX-1801 Scotchban Protector Film from Minnesota Mining and Mineral Company of St. Paul, Minnesota, are available which are microporous and thus breathable without the use of fillers. Generally to qualify as being "breathable" for the present application, the resultant film/nonwoven laminate should have a water vapor transmission rate of 300 grams per square meter per 24 hours or greater as defined by the test method described below.

Suitable polymers for forming the film layer 12 are any polymers or polymer blends which can be formed into films, stretch-thinned to an effective gauge of 0.01397 mm (0.55 mils) or less and then be bonded to a fibrous nonwoven support layer 14 without causing pinholes in the film layer which would permit the passage of liquids such as water, urine, blood and other body fluids. Polyolefin-based films have been found to work particularly well with respect to the present invention. For purposes of the present invention, a film is considered to be "polyolefin-based" if the polymer portion of the film, exclusive of any filler materials, has at least 50 weight percent polyolefin based upon the total weight of polymer in the film.

In addition to polymer, the film layer 12 may also include a filler to make the film breathable, more opaque or to impart some other property. As used herein a "filler" is meant to include particulates and other forms of materials which can be added to the film polymer extrusion blend and which will not chemically interfere with or adversely affect the extruded film but which are able to be uniformly dispersed throughout the film. Generally the fillers will be in particulate form and usually will have somewhat of a spherical shape with average particle sizes in the range of about 0.1 to about 7 $\mu$m. In addition, the film will usually contain at least 30 percent filler based upon the total weight of the film layer. Both organic and inorganic fillers are contemplated to be within the scope of the present invention provided that they do not interfere with the film formation process, the breathability of the resultant film or its ability to bond to the fibrous polyolefin nonwoven web or comfort layer. Examples of fillers include calcium carbonate ($CaCO_3$), various kinds of clay, silica ($SiO_2$), alumina, barium sulfate, sodium carbonate, talc, magnesium sulfate, titanium dioxide, zeolites, aluminum sulfate, cellulose-type powders, diatomaceous earth, magnesium sulfate, magnesium carbonate, barium carbonate, kaolin, mica, carbon, calcium oxide, magnesium oxide, aluminum hydroxide, pulp powder, wood powder, cellulose derivative, polymer particles, chitin and chitin derivatives.

An important aspect of the present invention is the ability to form a very thin film with sufficient strength in the machine direction and then couple it with a fibrous nonwoven support layer to yield a laminate which is low cost due to maximum reduction in materials and at the same time, sufficiently soft to the touch and strong so as to be suitable for uses such as the outer cover of a personal care absorbent article. Consequently, applicants have found that the film layer 12 must have an "effective" film thickness or gauge of 0.01397 mm (0.55 mils) or less. The effective gauge is used to take into consideration the voids or air space in breathable film layers. For normal non-filled/non-breathable films the actual gauge and the effective gauge of the film will most likely be the same. However, for filled films that have been stretched, the thickness of the film will also include the air spaces. In order to disregard this added volume, the effective thickness is calculated according to the test method set forth below.

Once a film has been stretch-thinned to an effective gauge of 0.01397 mm (0.55 mils) or less, it will tend to be "splitty." By splitty it is meant that the film will tend to split or tear along lines parallel to the machine direction (the direction of stretching), thereby making it weak from a tensile standpoint in the cross-machine direction. As a result, the film must be reinforced by some type of support layer 14. In addition, the overall laminate must be soft to the touch, at least on one side. Consequently, the film layer 12 is bonded to a layer of fibrous nonwoven web material generally having a fairly low basis weight such as 67.82 $g/m^2$ (two oz. per square yard (osy)) and even 33.91 $g/m^2$ (one osy) and less.

The support layer 14 can be any material which will provide a soft feel and a laminate with a ratio of cross-machine direction to machine direction Elmendorf tear strengths of less than 3.5. Fibrous nonwoven webs have been found to work particularly well due to their low cost, good strength, integrity and soft feel. Spunbond webs which have long essentially continuous fibers, once bonded and properly engineered, work well. The formation of spunbond webs is well-known. See for example patents such as Appel et al., US-A-4,340,563; Dorschner et al., US-A-3,692,618; Kinney, US-A-3,338,992 and US-A-3,341,394; Levy, US-A-3,276,944; Peterson, US-A-3,502,538; Hartman, US-A-3,502,763, Matsuki et al., US-A-3,802,817 and Dobo et al., US-A-3,542,615 all of which are incorporated herein by reference in their entirety.

Suitable polymers for forming a fibrous nonwoven web support layer include, but are not limited to, homopolymers, copolymers and blends of polyolefins and polyesters. In addition, the fibers may be single component fibers or multi-component fibers such as bicomponent fibers and biconstituent fibers. Biconstituent fibers are extruded from a homogeneous mixture of two different polymers. Such fibers combine the characteristics of the two polymers into a single fiber. Bicomponent or composite fibers are composed of two or more polymer types in distinct areas of the fiber such as in a side-by-side or sheath-core configuration.

Fiber size and basis weight can be varied, bearing in mind the requirements of softness and strength. The degree of bonding of the web will also affect these properties. Polymer selection will also affect softness. Generally, fiber sizes will be about 0.666 tex (about 6 denier) or less and basis weights will be about 67.82 $g/m^2$ (two oz. per square yard) or less. In heavy duty applications, the basis weights may be higher. Bonding of the fibers in the support layer 14 can be through the use of heat and/or pressure as with ultrasonic bonding equipment and heated bonding rolls. Point bonding has been found to create a softer nonwoven web than overall bonding with total bond area of 20 percent or less being desirable. Other types of bonding can include adhesive bonding such as with latexes, powdered or dry adhesives and solvent-based adhesives.

Bonding or lamination of the film layer 12 to the support layer 14 can be by any suitable means which will yield a laminate with sufficient MD strength as measured by its Elmendorf tear strength of 100 grams or greater and sufficient softness as measured by its cup crush value of less than 150 grams. In addition, where liquid barrier properties are important, the laminating process should not result in a laminate that will leak. In view of the foregoing requirements, possible laminating techniques or methods include, but are not limited to, ultrasonic bonding, adhesive bonding and thermomechanical bonding such as with heated and patterned laminating rolls. Lamination may take place across the entire interface between the two layers, however, extensive bonding tends to reduce the overall softness of the laminate 10. As a result, relative to a single side/surface of the laminate, it has been found to be more desirable to limit the total bond area relative to the surface area being measured to a maximum of 60 percent. Generally this can be accomplished by using a bond pattern having discrete bond points or by applying discrete sites of adhesion.

As described thus far, the laminate 10 has included a film layer 12 and a single support layer 14 laminated to one side of the film layer 12. If desired, it is also possible to laminate a second support layer 16 to a side of the film layer 12 opposite the first support layer 14. While not being necessary, it is most likely that the second support layer 16 will also be a fibrous nonwoven web. As a result, the options and features described previously with respect to the first support layer 14 can also be employed with respect to the second support layer 16. In addition, the same types of laminating techniques described above may be used in the lamination of the second support layer 16 to the film layer 12.

Creating a three-layer laminate in lieu of a two-layer laminate can provide certain advantages, especially with respect to certain end-use applications. Providing a second support layer 16 can improve the overall strength of the laminate 10, especially in the MD direction. It should be appreciated, however, that with increased overall basis weight for the laminate, cup crush values may increase though it is still possible to achieve peak load cup crush values of 300 grams or less.

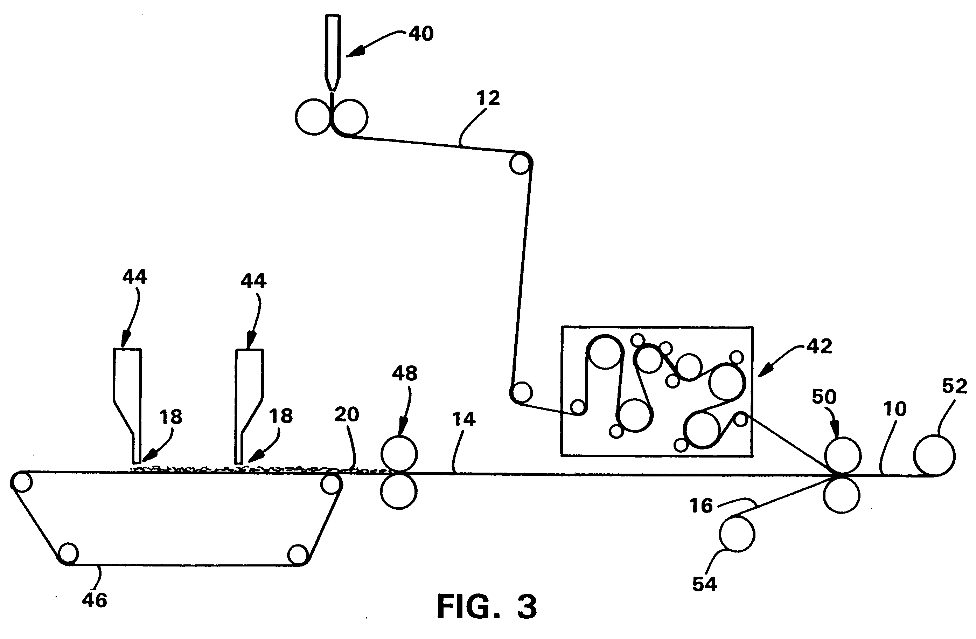

Referring to Figure 3, there is shown in schematic form, a process for forming a stretch-thinned film and nonwoven laminate according to the present invention. The film layer 12 is formed using any type of conventional film forming equipment 40 such as cast or blown equipment. As mentioned earlier, the film 12 so formed, if so desired, can contain a filler or other additive to impart particular properties to the film layer 12. After the film layer 12 has been formed, it is sent through a film stretching apparatus 42 to stretch and thin the film to an effective gauge of 0.01397 mm (0.55 mils) or less. One type of apparatus 42 suitable for such stretch-thinning is a Machine Direction Orienter (MDO) Unit, Model No. 7200 from the Marshall and Williams Company of Providence, Rhode Island.

While the film layer 12 is being formed and thinned, the fibrous nonwoven web support layer 14 is also being formed. Referring to Figure 3, a conventional fibrous nonwoven web forming apparatus 44, such as a spunbond machine, is used to form a support layer 14. The long, essentially continuous fibers 18 are deposited onto a forming wire 46 as an unbonded matt 20 and the unbonded matt is then sent through a pair of bonding rolls 48 to bond the fibers together and increase the tear strength of the resultant web 14. One or both of the rolls are often heated to aid in bonding. Typically, one of the rolls 48 is also patterned so as to impart a discrete bond pattern with a prescribed bond surface area to the web 14. The other roll is usually a smooth anvil roll but this roll may also be patterned if so desired.

Once the film layer 12 has been sufficiently thinned and oriented and the support layer 14 has been formed, the two layers are brought together and laminated to one another using a pair of laminating rolls or other means 50. As with the bonding rolls 48, the laminating rolls 50 may be heated. Also, at least one of the rolls is patterned to create a discrete bond pattern with a prescribed bond surface area for the laminate 10. Generally, the maximum bond point surface area for a given area of surface on one side of the laminate will not exceed about 60 percent of the total surface area.

After the laminate has been formed, it is wound into a roll 52 for subsequent processing. Alternatively, the laminate 10 may continue in-line for further conversion.

It should be noted that the process shown in Figure 3 may be altered in a number of ways without departing from the spirit and scope of the present invention. For example, a different apparatus 42 can be used for stretch-thinning the film layer 12. Different film/nonwoven web forming equipment 44 such as meltblown and bonded carded web equipment may be used in place of the spunbond equipment. In addition, other means for bonding and laminating the support layer 14 and laminate 10 may be used provided the resultant laminate 10 has the required properties described herein. Lastly, the formation processes for making the film and support layers may be done at a remote site and rolls of the two materials may be unwound into the process.

Referring again to Figure 3, the process shown also may be used to create a three layer laminate 10 such as is shown in Figure 2 of the drawings. The only modification to the previously described process is to feed a supply 54 of a second fibrous nonwoven web support layer material 16 into the laminating means 50 on a side of the film layer 12 opposite that of the first fibrous nonwoven web support layer 14. As shown in Figure 3, the supply of material for layer 16 is in the form of a preformed roll 54 of material. As with the other layers 12 and 14, layer 16 may be formed directly in-line or it may be preformed and then fed into the process from a supply roll. In either event, the second support layer 16 is fed into the laminating means 50 and is laminated to the film layer 12 in the same fashion as the first support layer 14.

## TEST METHODS

The properties of the present invention were determined using a series of test procedures. These properties included MD and CD Elmendorf tear strengths, cup crush which is related to softness, effective gauge and water vapor transmission rate. The test procedures and/or a reference to the published standards are set forth below. Three replications were performed for each of the test values given in the examples.

## Elmendorf Tear Strength

The Elmendorf tear strength is a measure of the average force required to propagate a single-nip, tongue-type tear starting from a cut in a fabric by means of a falling-pendulum (Elmendorf) apparatus. It can be measured in both the machine direction (MD) and cross-machine direction (CD) for both single layer materials and laminates. Samples were cut having dimensions of 102 mm by 63 mm. For cross-machine direction measurements, samples were cut with the long axis of the sample being parallel to the machine direction of the sample and thus the direction of orientation. For machine direction measurements, samples were cut with the long axis of the sample being perpendicular to the machine direction of the sample and thus the direction of orientation. In all cases, the initial cut in the samples was in the longer side of the specimens. As shown by the data below, the CD Elmendorf tear strengths were greater than the MD values. The test is performed in accordance with ASTM Standard Test Method D1424-83 except that the 6 mm deviation rule in section 11.5 of the test procedure was disregarded.

## Cup Crush

The cup crush of an individual layer or laminate was measured according to the following procedure and is shown in units of grams. The cup crush is an indication of the softness of a material and the lower the cup crush value, the softer the material. This procedure was conducted in a controlled environment wherein the temperature was about 22.78°C (about 73°F) and the relative humidity was about 50 percent. Samples were tested using a Material Test Instrument and Crush Test Stand available from Kimberly-Clark Corporation Quality Assurance Department in Neenah, Wisconsin. The Material Test Instrument and Crush Test Stand included a model 11 foot, a model 21 forming cylinder, a model 31 steel ring, a model 41 forming cup, a calibration set and an Epson FX-86e printer with cable.

The steel ring was placed over the forming cylinder and a 22.86 x 22.86 cm (9 x 9 inch) sample was centered over the forming cylinder. The forming cylinder was inserted into the forming cup until the sample was pinched between the forming cylinder and the steel ring all the way around the steel ring. The forming cup was placed on top of the cylinder plate of the load cell and firmly seated over the ridge of the cylinder plate. The foot was mechanically lowered into the forming cup, crushing the sample while the Materials Test Instrument measured the peak load needed to crush the sample.

## Water Vapor Transmission Rate

The water vapor transmission rate (WVTR) for the sample materials was calculated in accordance with ASTM Standard E96-80. Circular samples measuring 7.62 cm (three inches) in diameter were cut from each of the test materials and a control which was a piece of CELGUARD® 2500 film from Hoechst Celanese Corporation of Sommerville, New Jersey. CELGUARD® 2500 film is a microporous polypropylene film. Three samples were prepared for each material. The test dish was a number 60-1 Vapometer pan distributed by Thwing-Albert Instrument Company of Philadelphia, Pennsylvania. One hundred milliliters of water was poured into each Vapometer pan and individual samples of the test materials and control material were placed across the open tops of the individual pans. Screw-on flanges were tightened to form a seal along the edges of each pan, leaving the associated test material or control material exposed to the ambient atmosphere over a 6.5 cm diameter circle having an exposed area of approximately 33.17 cm$^2$. The pans were placed in a forced air oven at 37.8°C (100°F) for 1 hour to equilibrate. The oven was a constant temperature oven with external air circulating through it to prevent water vapor accumulation inside. A suitable forced air oven is, for example, a Blue M Power-O-Matic 60 oven distributed by Blue M Electric Company of Blue Island, Illinois. Upon completion of the equilibration, the pans were removed from the oven, weighed and immediately returned to the oven. After 24 hours, the pans were removed from the oven and weighed again. The preliminary test water vapor transmission rate values were calculated as follows:

$$\text{Test WVTR} = (\text{grams weight loss over 24 hours}) \times 315.5 \ \text{g/m}^2/24 \ \text{hrs}$$

The relative humidity within the oven was not specifically controlled.

Under predetermined set conditions of 37.8°C (100°F) and ambient relative humidity, the WVTR for the CELGUARD® 2500 control has been determined to be 5000 g/m² for 24 hours. Accordingly, the control sample was run with each test and the preliminary test values were corrected to set conditions using the following equation:

$$WVTR = (Test\ WVTR/control\ WVTR) \times 5000\ g/m^2/24\ hrs.)(g/m^2/24\ hrs)$$

Effective Gauge

The effective gauge of a material was calculated by dividing the basis weight of the film layer by the density of the polymer(s) and fillers forming the film.

The effective gauge of a layer of film was calculated by multiplying 0.001334 (a metric to English conversion factor) times the weight per unit area of the film sample in ounces per square yard and dividing the result by the density of the polymer formulation in grams per cubic centimeter to yield the effective gauge in inches.

Example I

In Example I a two layer, breathable stretch-thinned film and nonwoven laminate was made according to the present invention. The film layer contained, on a total weight percent basis, 65 percent English China Supercoat calcium carbonate ($CaCO_3$) with a 1 micron average particle size and a 7 μm top cut. The calcium carbonate was obtained from ECCA Calcium Products, Inc. in Sylacauga, Alabama, a division of ECC International. The calcium carbonate was blended with 20 percent by weight of linear low density polyethylene made from a blend of Dowlex® 2517 linear low density polyethylene and Dowlex® 2532 linear low density polyethylene blended in a weight ratio of 1:4 such that the melt index of the blend was 10 M.I. (/10 minutes at 87.78°C (190°F)). The Dowlex® polymers are available from Dow chemical U.S.A., Midland, Michigan. The remaining 15 percent by weight of the formulation comprised Himont KS051P polypropylene-based polymer from Himont, USA of Wilmington, Delaware. The KS051P polymer is an olefinic thermoplastic elastomer or TPO multistep reactor product wherein an amorphous ethylene propylene random copolymer is molecularly dispersed in a predominately semicrystalline high polypropylene monomer/low ethylene monomer continuous matrix. The film had an initial gauge of 0.0375 mm (1.5 mil) and was stretch-thinned to an effective gauge of 0.0105 mm (0.42 mils) using a machine direction orienter (MDO) unit of the type shown in Figure 3. The film was heated to a temperature of 76.67°C (170°F) and the film was run through the MDO unit at a line speed of 152.4 m (500 feet) per minute to stretch the film approximately four times its original length.

The first fibrous nonwoven web support layer was a 16.955 g/m² (0.5 oz. per square yard) spunbond web made from approximately 0.222 to 0.2778 tex (2.0 to 2.5 denier) polypropylene fibers. The polymer used to make the spunbond web was Exxon 3445 polypropylene from the Exxon Chemical Company of Houston, Texas. The spunbond web was prebonded using discrete bond points with a total bond area of 17 percent per unit area of web.

Lamination of the two layers was effected using a patterned laminating roll with a baby objects pattern at a temperature of 110°C (230°F) and a smooth anvil roll at a temperature of 71.11°C (160°F). The film layer was positioned against the anvil roll and the pressure was set at 137.895 kPag (20 pounds per square inch gauge (psig)). The resultant laminate had total bond area of 14 percent per unit area and a basis weight of 38.3 g/m² (1.15 osy).

Testing of the laminate indicated that the stretch-thinned film layer by itself had a peak load cup crush value of 75 g, a CD and MD Elmendorf tear strength of 144 and 32 g, respectively. The ratio of the CD to MD Elmendorf tear strength was 4.5. The laminate had a peak load cup crush of 76 g. Elmendorf tear strengths for the laminate in the CD and MD directions were 496 and 224 g respectively and the CD/MD ratio was 2.2. Lastly, the laminate had a water vapor transmission rate of 3700 g/m² per 24 hours.

Example II

In Example II a two-layer non-breathable, stretch-thinned film and nonwoven laminate was made according to the present invention. The film layer comprised on a weight percent basis, based upon the total weight of the film, 19 percent titanium dioxide ($TiO_2$) concentrate, 32 percent polypropylene, 5 percent low density polyethylene and 32 percent of a polypropylene-based polymer designated Himont KS080 Catalloy polymer from Himont, USA of Wilmington, Delaware. The titanium dioxide was Ampacet 110310 from Ampacet Corporation of Tarrytown, New York. The polypropylene was Exxon 3445 polypropylene from Exxon Chemical Company of Houston, Texas and the low density polyethylene was Quantum NA334 low density polyethylene from Quantum Chemical Corporation of New York, New York. The film had an initial gauge of 0.015 mm (0.6 mils) and was stretch-thinned to an effective gauge of 0.01 mm (0.41 mils). During the stretch-thinning process the film was heated to a temperature of 87.78°C (190°F) and the film was stretched to approximately two times its original length at a line speed of 91.44 m (300 feet) per minute. The film layer by itself had a peak load cup crush value of 36 g and MD and CD Elmendorf tear strengths of 16 and 320 g, respectively. The fibrous nonwoven web support layer was the same as that used in Example I. Lamination conditions and lamination bond area were the same as in Example I except for the laminating pressure which was 172.37 kPag (25 psig).

The resultant laminate had a basis weight of 26.8 g/m² (0.8 osy), a peak load cup crush value of 104 g, a CD Elmendorf tear strength of 352 g and a MD Elmendorf tear strength of 160 g. The ratio of the CD to MD Elmendorf tear strengths was 2.2.

Example III

In Example III, a three layer, stretch-thinned film and nonwoven laminate was made according to the present invention. The first and second fibrous nonwoven web support layers were the same 16.95 g/m² (0.5 osy) spunbond web material used in Examples I and II and the film layer was made from the same film material with the same 0.0105 mm (0.42 mil) effective gauge as was used in Example I. Lamination of the three layers, with the film in the middle, took place using the same patterned and anvil rolls as were used in Examples I and II. As a result, total lamination bond area per unit area of either support layer was 14 percent. Lamination took place at a line speed of 30.48 m (100 feet) per minute with both the patterned and anvil rolls heated to a temperature of 112.78°C (235°F) and with the lamination pressure between the rolls set at 206.8 kPag (30 psig).

The resultant laminate had a basis weight of 53.6 g/m² (1.6 osy), a water vapor transmission rate of 3700 g/m² per 24 hours and a peak load cup crush value of 140 g. CD and MD Elmendorf tear strengths for the laminate were 640 and 224 g, respectively, and the ratio of CD to MD Elmendorf tear strengths was 2.9.

The stretch-thinned film and nonwoven laminate of the present invention has a wide variety of uses and applications. Non-restrictive examples of such uses and applications include body side liners and outer covers for personal care absorbent products. Typically such products include a body side liner which faces the user and an outer cover which forms the external surface of the product. Disposed between these two materials there is usually an absorbent core for absorbing body exudates such as blood, urine, feces and other body fluids. The material of the present invention may be used to form all or a portion of either or both of the body side liner and the outer cover.

Articles of clothing also sometimes employ films and/or nonwovens in their construction. As a result, the material of the present invention may be used to form all or a portion of such articles.

In the health care arena many surgical drapes, gowns and sterile wraps are made from films and/or nonwovens. Consequently, the material of the present invention may be used to form all or a portion of any one of these products as well.

Other areas of possible applications for the material of the present invention include, but are not limited to, packaging materials, shower curtains, tent material and covers for such items as furniture, computers, automobiles and other vehicles.

Having thus described the invention in detail, it should be appreciated that various modifications and changes can be made to the present invention without departing from the spirit and scope of the following claims.

**Claims**

1. A process for forming a stretch-thinned film and nonwoven laminate (10) comprising:

   a) sufficiently stretching a polyolefin-based film (12) to cause said film (12) to have an effective gauge of 0.01397 mm (0.55 mils) or less and a cross-machine direction to machine direction ratio of Elmendorf tear strength of 3.5 or greater, and
   b) bonding a first fibrous nonwoven web support layer (14) to said film (12) to form a laminate (10).

2. The process of claim 1 wherein said bonding creates a plurality of discrete bond points between said film (12) and said first fibrous nonwoven web support layer (14) with a maximum total bond area of about 60 percent.

3. The process of one of the preceding claims which further includes bonding a second fibrous nonwoven web support layer (16) to a surface of said film opposite said first fibrous nonwoven web support layer (14).

4. A stretch-thinned film and nonwoven laminate (10) comprising:
   a polyolefin-based film layer (12) having an effective gauge of 0.01397 mm (0.55 mils) or less, a ratio of cross-machine direction to machine direction Elmendorf tear strengths of 3.5 or greater, and
   a first fibrous nonwoven web support layer (14) bonded to said film layer (12).

5. The stretch-thinned film and nonwoven laminate (10) of claim 4 wherein said film layer (12) is bonded to said first fibrous nonwoven web support layer (14) at a plurality of discrete bond points with a maximum total bond area of about 60 percent.

6. The stretch-thinned film and nonwoven laminate (10) of claim 4 or 5 wherein a second fibrous nonwoven web support layer (16) is bonded to a surface of said film layer (12) opposite said first fibrous nonwoven web support layer (14).

7. The stretch-thinned film and nonwoven laminate (10) of any one of claims 4 to 6 wherein said laminate has a minimum machine direction Elmendorf tear strength of 100 grams or greater and a peak load cup crush value of less than 150 grams.

8. The stretch-thinned film and nonwoven laminate (10) of any one of claims 4 to 6 wherein said laminate has a peak load cup crush value of 300 grams or less.

9. The stretch-thinned film and nonwoven laminate (10) of any one of claims 4 to 8 wherein said laminate has a water vapor transmission rate of 300 grams/meter$^2$/24 hours or greater.

10. The stretch-thinned film and nonwoven laminate (10) of any one of claims 4 to 9 wherein said film contains at least 30 percent filler based upon the total weight of said film layer.

11. The stretch-thinned film and nonwoven laminate (10) of any one of claims 4 to 10 wherein said laminate (10) has a ratio of cross-machine direction to machine direction Elmendorf strengths of less than 3.5.

12. A personal care absorbent product comprising a body side liner, an outer cover and an absorbent core disposed between said body side liner and said outer cover, at least one of said body side liner and said outer cover comprising the stretch-thinned film and nonwoven laminate (10) of any one of claims 4 to 11.

13. An article of clothing wherein at least a portion of said article comprises the stretch-thinned film and nonwoven laminate of any one of claims 4 to 11.

14. A surgical drape wherein at least a portion of said drape comprises the stretch-thinned film and nonwoven laminate of any one of claims 4 to 11.

15. A sterile wrap wherein at least a portion of said wrap comprises the stretch-thinned film and nonwoven laminate of any one of claims 4 to 11.

FIG. 1

FIG. 2

FIG. 3

European Patent
Office

**EUROPEAN SEARCH REPORT**

Application Number

EP 95 10 8644

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP-A-0 323 629 (IDEMITSU PETROCHEMICAL CO. LTD., NESTE OY)<br>* page 6, line 6 - page 7, line 23; claims 9,16; example 4; table 1 *<br>--- | 1-4,6,9, 10,12-15 | B32B31/04<br>B32B5/02<br>B32B27/32<br>B29C55/00<br>A61B19/08 |
| Y<br>A | EP-A-0 309 073 (EXXON CHEMICAL PATENTS INC.)<br>* page 3, line 13 - page 5, line 55 *<br>* page 9, line 1 - line 15; claims 1,3,6-10 *<br>--- | 1,4,9, 10,12-15<br>7,8,11 | A61F13/15 |
| Y<br>A | EP-A-0 084 903 (THE PROCTER & GAMBLE COMPANY)<br>* claims 1-10 *<br>--- | 2<br>5 | |
| Y | EP-A-0 302 597 (TONEN SEKIYUKAGAKU K.K.)<br>* page 3, line 25 - page 4, line 38; claims 1,6; figure 2 *<br>--- | 3,6 | |
| Y | EP-A-0 409 567 (TONEN SEKIYUKAGAKU KABUSHIKI KAISHA)<br>* claims 1-8; figure 2 *<br>--- | 3,6 | TECHNICAL FIELDS SEARCHED (Int.Cl.6) |
| A | GB-A-2 115 702 (KAO CORPORATION)<br><br>* page 2, line 23 - line 44 *<br>* page 16, line 24 - page 17, line 27; claims 1-5,7-11; figures 2,3 *<br>--- | 1,4,7,9, 10,12 | B32B<br>A61F<br>A61B |
| A | DATABASE WPI<br>Week 8636<br>Derwent Publications Ltd., London, GB;<br>AN 86-236752<br>& JP-A-61 167 550 (TOYO CHEMICAL KK) , 29 July 1986<br>* abstract *<br>--- | 1,4,11 | |

-/--

The present search report has been drawn up for all claims

| Place of search | Date of completion of the search | Examiner |
|---|---|---|
| THE HAGUE | 25 October 1995 | Van Nieuwenhuize, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................

& : member of the same patent family, corresponding document

EPO FORM 1503 03.82 (P04C01)

**European Patent Office**

## EUROPEAN SEARCH REPORT

Application Number

EP 95 10 8644

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP-A-0 066 672 (MITSUBISHI CHEMICAL INDUSTRIES LIMITED) <br> * page 14, line 17 - line 22; claims 1,10 * | 1,4, 12-15 | |
| A | DATABASE WPI <br> Week 8849 <br> Derwent Publications Ltd., London, GB; <br> AN 88-351188 <br> & JP-A-63 264 336 (ASAHI CHEMICAL IND KK) <br> , 1 November 1988 <br> * abstract * | 1,4,13 | |
| A | DATABASE WPI <br> Week 8120 <br> Derwent Publications Ltd., London, GB; <br> AN 81-35328d <br> & JP-A-56 032 241 (MITSUI TOATSU CHEM INC) <br> , 1 April 1981 <br> * abstract * | 2,5 | |
| A | US-A-4 348 444 (ALAN D. GRAIG) <br> * column 3, line 1 - column 4, line 60; claim 8; figure 4 * | 3,6 | TECHNICAL FIELDS SEARCHED (Int.Cl.6) |

The present search report has been drawn up for all claims

| Place of search | Date of completion of the search | Examiner |
|---|---|---|
| THE HAGUE | 25 October 1995 | Van Nieuwenhuize, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)